# EUROPEAN PATENT SPECIFICATION

(11) **EP 2 683 745 B1**
(45) Date of publication and mention of the grant of the patent: **16.05.2018**
(21) Application number: 12707495.3
(22) Date of filing: 06.03.2012
(51) Int. Cl.: C08B 30/14

(54) **A POWDERED ACID-LOADED CARRIER MATERIAL**
SÄUREBELADENE QUELLSTÄRKE
AMIDON PREGELATINISE CHARGÉES D'ACIDE

(30) Priority: 09.03.2011 EP 11001929
(43) Date of publication of application: 15.01.2014
(73) Proprietor: Cargill, Incorporated, Wayzata, MN 55391 (US)
(72) Inventor: VEELAERT, Sarah, B-1980 Eppegem (BE)
(74) Representative: Morariu Radu, Mihai Dorin
(86) International application number: PCT/EP2012/001010
(87) International publication number: WO 2012/119765

(56) References cited:
- WO-A1-2009/103514
- WO-A1-2010/046038
- DE-C1- 3 506 513
- GB-A- 1 249 250
- GB-A- 1 419 996
- GB-A- 2 112 621
- US-A- 3 657 010
- US-A- 3 955 009
- US-A- 4 022 924
- US-A- 4 081 567

## Description

### Field of the Invention

The present invention concerns a powdered acid-loaded starch material comprising one or more liquid acidic components and a solid carrier material being pregelatinised starch selected from pregelatinised starch, puffed starch and one or more water soluble liquid acids absorbed into and/or onto said solid carrier material. The present invention further concerns processes for preparing said powdered acid-loaded starch material. In addition, the present invention relates to the use of said powdered liquid-loaded starch material in food and animal feed products, pharmaceuticals, nutraceuticals, agrochemicals, and cosmetic or personal care products and the like and more specifically a snack flavoured with the powdered acid-loaded starch material.

### Background of the Invention

The prior art is replete with controlled delivery systems for the delivery of flavours, perfume and fragrances, drugs, and other active ingredients for cleaning, health and skin, and the like, mainly hydrophobic materials.

Delivery systems for the delivery of flavours essentially exist in 2 forms. One form is a suspension or solution which, depending on the level of flavours, can be pasty or liquid. Although these suspensions are often useful, they may have some disadvantages such as ease-of-handling, limited number of applications, and limited shelf life.

Another form is a particulate delivery system. Such a system is sometimes preferred because it's easier to handle.
One technique for making such particulate delivery systems is the adsorption on a carrier. This technique is often referred to as plating. Plating is the old flavour terminology for the adsorption of a liquid flavour on a fine powder and is the oldest method to transform a liquid flavour into powder. Salt, sugar, maltodextrins and starches are commonly used as carriers. The process is a physical action of solid-liquid intersurface tension and surface adsorption.

WO2009/103514 provides a liquid-loaded starch material comprising a solid carrier material consisting of pregelatinised, non-granular starch material which consists of flake-shaped starch particles, wherein the size distribution of the starch particles is such that at least 50% by weight of the starch particles have a particle size of between 100 and 375 µm, and wherein the BET specific surface area is less than or equal to 0.5 m²/g and one or more liquid components.

WO2010/046038 concerns a puffed popcorn starch containing powder having a loose bulk density of less than 120 g/L and a particle size smaller than 1 mm, a non-aqueous liquid-loaded starch material comprising said powder.

Although there are various starch-based materials available in the art, which are used as carriers or encapsulating materials for liquids, solids and volatile substances, there is still a need in the industry for additional starch-based solid carrier materials having a high loading capacity for water soluble liquid acidic components, which can be prepared in a simple and cost-efficient manner and which result in a powdered acid-loaded carrier material.

### Summary of the Invention

The present invention relates to a powdered acid-loaded carrier material, comprising one or more water-soluble liquid acidic components and a solid carrier material being pregelatinised starch selected from the group consisting of pregelatinised non-granular starch material, pregelatinised granular starch, partially gelatinised starch, puffed starch and mixtures of two or more thereof, and wherein the water-soluble liquid acidic component is absorbed into and/or onto said solid carrier material.

The present invention relates to a process for preparing the powdered acid-loaded starch material according the present invention and is comprising the steps of:
a. providing a solid carrier material being pregelatinised starch selected from the group consisting of pregelatinised non-granular starch material, pregelatinised granular starch, partially gelatinised starch, puffed starch and mixtures of two or more thereof,
b. loading one or more water-soluble liquid acidic components into and/or onto the solid carrier material provided in step (a) by applying one or more water-soluble liquid acidic components to the carrier material under agitation.

Furthermore, the current invention relates to the use of a powdered acid-loaded carrier material according to the present invention for use in food and animal feed products, pharmaceuticals, nutraceuticals, agrochemicals, cleaning agents, home care products, industrial products, and cosmetic or personal care products and cosmetic or personal care products.

The current invention further relates to a snack flavoured with the powdered acid-loaded carrier material according to the present invention, and wherein the powdered acid-loaded carrier material is present in an amount of 0.5-10%.

### Detailed Description

The present invention relates to a powdered acid-loaded carrier material, comprising one or more water-soluble liquid acidic components and a solid carrier material being pregelatinised starch selected from the group consisting of pregelatinised, non-granular starch material, pregelatinised granular starch, partially gelatinised starch, puffed starch and mixtures of two or more thereof, and wherein the water-soluble liquid acidic component is absorbed into and/or onto said solid carrier material.

The powdered acid-loaded carrier material is preferably substantially dry. More preferably, it is a dry powdered acid-loaded carrier material such that it can behave as a free flowing powder which improves e.g. the handling of it.

### Starch Carrier

Starch is a polysaccharide that is produced in the form of granules in most plant cells. Such starch granules consist of highly ordered crystalline regions and less organized amorphous regions. When present in this granular state, the starch is referred to as "native starch".

Suitable starch containing kernels refer to corn, pea, potato, sweet potato, sorghum, banana, barley, wheat, rice, sago, amaranth, tapioca, arrowroot, canna, and low amylose (containing no more than about 10% by weight amylose, preferably no more than 5%) or high amylose (containing at least about 40% by weight amylose) varieties thereof. Also suitable are starches derived from a genetically modified starch crop. A preferred starch for use herein has an amylose content below 40%, including waxy corn starch with less than 1% amylose content. Particularly preferred starches include rice, wheat, tapioca, corn, and potato starches, in particular popcorn (maize) starch.

The starch may be chemically modified, be modified by heat treatment or by physical treatment. The term "chemically modified" or "chemical modification" includes, but is not limited to, crosslinked starches, starches modified with blocking groups to inhibit retrogradation, starches modified by the addition of lipophilic groups, acetylated starches, hydroxyethylated and hydroxypropylated starches, inorganically esterified starches, cationic, anionic and oxidized starches, zwitterionic starches, starches modified by enzymes, and combinations thereof.

The preferred modification process is the pregelatinisation of starch, which is the collapse or disruption of molecular orders within starch granules, manifested in irreversible changes in properties such as granular swelling (penetration of water, which results in an increased randomness in the granular structure), native crystallite melting (decrease of crystalline regions of the starch granules due to the penetration of water, also called non-semi crystalline), loss of birefringence, and starch solubilization. Such pregelatinised starches are substantially soluble (swelling) in cold water without cooking and develop viscosity immediately (instant starches), in contrast to native starches. Pregelatinised starches are typically prepared by thermal, chemical, or mechanical processes. The particular process employed strongly affects the physical properties of the pregelatinised starches, in particular wettability, dispersibility and peak viscosity in cold water. Thermal processes are widely used as heat causes the conversion of crystalline regions into amorphous region, thereby promoting the penetration of water and swelling of the granules. Typical thermal processes to effect gelatinisation include spray-drying, roll-drying or drum-drying, extrusion, and other heating/drying processes. Depending on the method used and the specific process parameters employed, the produced pregelatinised starches may have lost or maintained their granular structure. The non-granular pregelatinised starches, typically prepared by roll-drying, drum-drying, extrusion and, in some cases, spray-drying, are widely used in various technical fields (see, e.g., U.S. patents Nos. 3,607,394 and 5,131,953). For some applications, however, granular pregelatinised starches are preferentially used because the intact granular structure imparts certain properties, such as improved texture. These granular pregelatinised starches may be prepared by, for example, specific spray-drying processes, which cause swelling and pregelatinisation while preventing destruction of the granule shape, or heating in aqueous organic solvents, such as alcohol-water mixtures, followed by drying (see, e.g., U.S. patents Nos. 4,465,702 and 5,037,929).
Pregelatinised starches are widely used in various technical fields to alter the viscosity or texture of a given product without requiring heating. For this reason, for example, numerous food products contain pregelatinised starches. Another important field of application is the pharmaceutical industry, where pregelatinised starches are traditionally used as a binder, filler or disintegrant, and to enhance drug stability and control release rates in modified-delivery dosage forms.

One preferred pregelatinised starch is a pregelatinised, non-granular starch material consisting of flake-shaped starch particles as described in WO2009/103514.
Preferably the pregelatinised, non-granular starch material consisting of flake-shaped starch particles as described hereinabove is produced by a roll-drying or drum-drying process. Roll-drying as well as drum-drying involve the heating of an aqueous starch slurry or paste to gelatinize the starch and to instantaneously remove the moisture. The aqueous starch slurry or paste may be first heated and subsequently dried or, more preferably, the starch may be simultaneously gelatinised by heating and dried using a commercially available drum-dryer or roll-dryer apparatus. As used herein, the term "roll-drying" refers to a process where an aqueous starch slurry or paste is cooked or partially cooked and passed on heated rolls (sometimes also referred to as "drums") for drying or, preferably, a process where the aqueous starch slurry or paste is simultaneously cooked and dried on heated rolls. The term "drum-drying", when used herein, refers to a process very similar to the roll-drying process, except that a thicker coating of the starch slurry or paste is applied to heated drums.
The present invention relates to a powdered acid-loaded carrier material wherein the pregelatinised, non-granular starch material is consisting of flake-shaped starch particles, wherein the size distribution of the starch particles is such that at least 50% by weight of the starch particles have a particle size of between 100 µm and 375 µm, and preferably wherein the BET specific surface area is less than or equal to 0.5 m²/g.

Alternatively, the starch maybe partially gelatinised. Partially gelatinised starch is consisting of granules pregelatinised to a gelatinisation degree of between 30% to 90%, preferably between 50% to 77%, more preferably between 55% to 77%, most preferably between 60% and 75%. The gelatinisation degree can be determined by measuring melting enthalpy by Differential Scanning Calorimetry (DSC), according to the method described by Remon in Int. J. of Pharmaceutics 56, (1989), 51-63. There exists a linear relationship between the melting enthalpy (i.e. gelatinisation energy) and the degree of gelatinisation. The difference between the melting enthalpy of uncooked starch and partially pregelatinised starch gives a measure for the degree of gelatinisation.
The gelatinisation degree is further determined by measuring residual birefrigence by microscopic analysis. The microscopic analysis of the partially pregelatinised starch is made with an optical microscope (Zeiss). Starch is dispersed into cold water at a concentration of 5% dry substance. A droplet of this solution is put on a microscopy glass, and covered by a fine glass. The sample is observed under polarised light. Pregelatinised starch is characterised by the absence of birefringence, while partially gelatinised starch is characterised by weakening of the birefringence.
Typically these partially gelatinised starches can be prepared by extrusion and/or superheated steam treatment.

Another pregelatinised starch carrier suitable in the present invention is a puffed starch containing powder, which is obtained from a puffed starch containing material wherein "puffed" refers to the well-known definition of puffing which indicates a swelling through a release of vapour in a puff. A puffed starch containing material is a swollen and/or burst kernel, which is containing starch in an amount of more than 30%, preferably more than 50% (between 60 and 70% for popcorn). One preferred puffed starch containing powder is described in WO2010/046038.

There are two traditional types of equipments used for puffing of starch: high temperature ovens or towers, or puffing guns.
Oven puffing is based on a sudden application of heat at atmospheric pressure so that the water in the kernel is vaporized in situ, thereby expanding the product. Suitable equipment for preparing the puffed starch containing kernel of the present invention are commercially available industrial ovens, for example conventional ovens, microwave ovens, dextrinizers, fluidized bed reactors and driers, mixers and blenders equipped with heating devices and other type of heaters, contact heating on e.g. metal surface, hot air puffing in a fluidized bed operation, turboreactors and the like. Possible heat transfer media include hot air, oil or superheated steam.
Gun puffing is based on a sudden transfer of kernels containing superheated steam from a high pressure to a low-pressure medium thereby allowing the water to suddenly vaporize and cause expansion. The advantage of the puffing gun or chamber is that they allow the puffing to be controlled and to be performed with a wide variety of kernels.

Degree of expansion of the kernel depends on the genotype, kernel size, water content of the starch containing material and/or processing conditions.
The preferred temperature is normally between 200 and 400 °C. The optimum moisture content of the kernel ranges from 10 to 20 %, preferably from 13 to 15%.

In a preferred embodiment of the present invention, a grinding step is applied in a mill over a sieve with a 1 to 2 mm sieve opening. It may be important to have cutting effect and avoid that porous structure is being disturbed and sealed during the grinding operation and the heat generation has to be minimized as well. Optionally pre-milling step without or with a coarser sieve opening, will be beneficial to have a primary size reduction and more efficient milling. Typical mills that could be used are cross beater mills, cutting mills, impact mills. The resulting ground kernel (= puffed popcorn starch containing powder) has a certain particle size distribution, preferably with more than 90% of the weight of the material below 1mm (passing a 1 mm sieve), and more than 70% below 0.5 mm.

Once the powder is ground (milled), it can be further fractionated (=separated) into different fractions by sieving, electrostatic separation or wind sifting. The advantage of wind-sifting obviously is that fractions with a lower bulk density can be easily isolated. With these separation steps it is also possible to remove the insoluble yellow and/or brown fibers, resulting from the pericarp of the raw material used. Especially when puffing occurs at too high temperatures these fibers are brown in nature and can be a potential disadvantage for certain applications such as milk replacement.
The present invention relates to a powdered acid-loaded carrier material wherein the puffed starch is having a loose bulk density of less than 150 g/l, preferably less than 120 g/l, more preferably less than 100 g/l.

Alternatively, a quite different starch carrier is a porous starch, which is not pregelatinised. The term "porous starch" as used herein means starch or starch granules having been modified by a substrate, preferably an enzyme, resulting in the structural lattice of the granule having holes, pores or openings which allow smaller molecules to enter the interstices of the starch granules. The starch granules suitable for modification and for use in the present invention may comprise any starch which is capable of being modified to increase pore volume or surface area, for example, corn or potato starch. An example of porous starch granules suitable for use in the present invention are starch granules modified by treatment, usually by amylase enzymes, to increase the pore volume and thereby producing a microporous starch matrix. Any of a wide variety of art-recognized alpha-amylase or glucoamylases including those derived from Rhizopus niveus, Asperigillus niger, and Rhizopus oryzae and Bacillus subtilis and alpha-amylases and glucoamylases of animal origin, can be used. Microporous starch granules prepared by the action of acid or amylase on granular starch are well known in the literature, see for example, Starch Chemistry and Technology, Whistler, Roy L., 2nd Edition, (1984), Academic Press, Inc. New York, N.Y.

These methods and others, as well as those disclosed herein, are suitable for preparing a partially hydrolyzed porous starch matrix. The duration of enzyme treatment necessary to produce microporous starch matrices suitable for use in accordance with this invention depends on a number of variables, including the source of starch, species and concentration of amylases, treatment temperature, and pH of the starch slurry. The progress of starch hydrolysis can be followed by monitoring the D-glucose content of the reaction slurry.
When the starch carrier is the porous starch, the carrier has an average particle size of from 0.5 micrometers to 400 micrometers, or the porous starch carrier has an average particle size of from 1 micrometers to 200 micrometers or from 2 micrometers to 100 micrometers. Finally, the porous starch carrier has an average particle size of from 10 micrometers to 50 micrometers.

Likewise as the porous starch, cyclodextrins are however excluded from the present invention. They are expensive in manufacturing and use.

### Acidic component

The acidic component is water soluble and is selected from the group consisting of acetic acid, formic acid, propionic acid, sulphuric acid, citric acid, ascorbic acid, salycilic acid, malic acid, sorbic acid lactic acid, tartaric acid, phosphoric acid, fumaric acid, leavening acids and mixtures of two or more thereof.

The acidic component is provided in a liquid form. Preferably it is provided in the form of a concentrated aqueous solution of acid wherein the solution is containing less than 10%, preferably less than 5%, more preferably less than 1% water, or in the form of a non-aqueous solution containing ethanol, methanol and the like, or in the form of a melted acid. This avoids the effect of water on the powder characteristics of the starch carrier, for instance the pregelatinised starch carrier starts to swell in presence of water. It was surprisingly found that due to applying concentrated water soluble liquid acids or water soluble molten acids, it is still feasible to obtain free-flowing powder loaded with water-soluble acids. The absence of water or the low amount of water avoids having swelling of the pregelatinised starch.

The present invention relates to a powdered acid-loaded carrier material wherein the one or more acidic components that is absorbed into and/or onto the solid carrier material constitute at least 10% by weight of the total weight, preferably at least 30%, more preferably at least 50% by weight of the powdered acid-loaded carrier particles.

### Process for preparing a powdered acid-loaded

The current invention relates to a process for preparing the powdered acid-loaded starch material of the current invention and the process is comprising the steps of:
a. providing a solid carrier material being pregelatinised starch selected from the group consisting of pregelatinised non-granular starch material, pregelatinised granular starch, partially gelatinised starch, puffed starch and mixtures of two or more thereof,
b. loading one or more water-soluble liquid acidic components into and/or onto the solid carrier material provided in step (a) by applying one or more water-soluble liquid acidic components to the carrier material under agitation.

For loading the starch material with one or more water-soluble liquid acidic components, the starch material may be placed in a vessel supporting mechanical mixing and preferable capable of being sealed. Suitable mixing devices are, for example, a paddle mixer, a ribbon blender, a V-blender, or a plough blade mixer. The one or more water-soluble acidic components are then supplied, for example poured, pumped or, preferably, sprayed via a nozzle, into the vessel and applied onto the agitated starch material. Spraying via a nozzle is advantageously used because the nozzle leads to the formation of small droplets that are more easily absorbed by the starch carrier material. Loading from the gas phase or under supercritical conditions is also possible. The mixing is continued until an even distribution of the acidic material into and/or onto the solid carrier is obtained. The time required for spraying or pumping is dependent upon the addition level of the acidic components onto the starch material and the time required to ensure complete absorption to form a free flowing powder.

Another suitable method for loading one or more water-soluble liquid acidic components onto the starch material may be a fluidized-bed loading process. In such a process, the carrier, i.e. the starch material is fluidized by forcing air or another gas upward through a bed of starch particles. The water-soluble liquid acidic components are then sprayed via a nozzle onto the fluidized starch particles to yield an acid-loaded starch material of evenly loaded starch particles.

A further suitable loading method for use herein comprises the steps of suspending the starch carrier material in the water-soluble liquid acidic components, followed by separating the powdered acid-loaded starch material from the acidic components by conventional separation methods, such as filtration or centrifugation.

Depending on the type of water-soluble liquid acidic component to be loaded, the acidic component may be heated or cooled. In case of high viscous liquid components, for example, it might be favourable to heat the acidic components to decrease the viscosity and facilitate the loading process. In case of temperature-sensitive acidic components, cooling might be desired or required. Means for effecting cooling or heating, such as a cooled or heated blender, are well-known to a person skilled in the art. The acids having a melting point above room temperature need to be heated to bring them into a liquid state to enable absorption by and migration into the starch carrier.

After having loaded the starch material with one or more water-soluble liquid acidic components, further processing steps may optionally follow. For example, flowing or anti-caking agents may be added to the liquid-loaded starch material, such as tricalcium phosphate, silica, silicates and/or stearates, to increase flowability. The powdered acidic-loaded starch material of the present invention may also be provided with a coat and/or further encapsulated by any suitable encapsulating or coating materials, such as maltodextrins, starches, modified starches, dextrins, oils, fats, waxes, hydrocolloids, proteins, as known in the art.

Optionally, also a drying and/or sieving step may be performed.

### Use

The powdered acidic-loaded starch material of the present invention may be incorporated into numerous different formulations, such as powders, granules, tablets, capsules, lozenges, pastes, gels, creams, salves, ointments, lotions, and the like. Preferred end-use applications of the powdered acidic-loaded starch material of the present invention include, but are not limited to, food and animal feed products, pharmaceuticals, nutraceuticals, agrochemicals, such as herbicides, pesticides, and fertilizers, cleaning agents, home care products, industrial products, cosmetic and personal care products, such as dry hair care products, shampoos, conditioners, antiperspirants, deodorants, mouthwashes, soaps, cosmetic and skin care creams, and the like.

In a preferred embodiment of the present invention, the powdered acidic-loaded starch material of the present invention is used in food products including, but not limited to, snacks, instant soups, seasoning, sauces, dry mixes for sauce preparations, dressings, marinades, dips, meat products , bakery products, baking powders, confectionery, dairy products, dried and/or freeze-dried vegetables.

The present invention further relates to a snack flavoured with the powdered acid-loaded carrier material of the present invention, and wherein the powdered acid-loaded carrier material is present in an amount of 0.5-10%.

The present invention will now be further illustrated and explained by reference to the examples given below.

### Examples

### Example1:

50 g flake shaped pregelatinised corn starch (Starrier™ R, Cargill) with an average particle size of 230 µ was loaded with 50 g glacial acetic acid (99,85% from Brenntag NV). The loading was done by adding the liquid acetic acid to the starch in a glass beaker followed by mixing with a spoon until a homogeneous powder mixture was obtained. The resulting acid loaded powder contained 50% acetic acid and consisted of individual agglomerates without effect of caking .

### Comparative Example 2:

A slurry of regular corn starch (18%) in water was spray dried via a bi-fluid nozzle with steam of 7.7 barg. The starch slurry was preheated to 72 °C before entering the nozzle. The temperature in the spray-drying chamber was 230 °C.
10 gram of this starch was mixed with 5 g of glacial acetic acid following the same procedure as described in example 1.

The resulting acid loaded powder contained 33% acetic acid, but started to show lumping.

### Example 3:

Pop corn was popped, milled and the resulting flour was air fractionated into different fractions. The low density fraction of the pop corn flour (< 100 g/liter) was used as a carrier in this experiment. 5 g of this low density pop corn flour was mixed with 20 to 30 g glacial acetic acid. The resulting acid loaded powder contained 80 resp. 86 % acetic acid, while no caking or lumping of the final powder was observed.

### Comparative Example 4:

Porous starch was obtained by treatment with α-amylase in a slurry reaction at 53 °C and a pH of 6 for 24 hours. 20 g of this porous starch was loaded with 15 g of glacial acetic acid. The resulting powder contained 43 % acetic acid. The powder was agglomerated without showing caking.

### Comparative Example 5:

50 g flake shaped pregelatinised corn starch (Starrier™ R, Cargill) with an average particle size of 230 µ was loaded with 13 g lactic acid (99% (L+) lactic acid from Fluka). The lactic acid crystals were heated in a glass beaker to temperature of 55 °C to obtain a liquid lactic acid solution. This solution was loaded on the starch as described in example 1.
The final powder contained 21 % lactic acid and was still behaving as a powder.

### Example 6:

The acid loaded powder obtained in example 1 was used to flavour potato chips. 100 g commercial potato chips were heated in an oven at a temperature of 80 °C. The chips were removed from the oven and 5 g of the acid loaded powder was immediately sprinkled over the hot chips and gently blended. The resulting chips had a clear vinegar taste.

## Claims

1. A powdered acid-loaded carrier material, comprising one or more water-soluble liquid acidic components and a solid carrier material being a pregelatinised starch selected from the group consisting of pregelatinised non-granular starch material, pregelatinised granular starch, partially gelatinised starch, puffed starch and mixtures of two or more thereof, and wherein the water-soluble liquid acidic component is absorbed into and/or onto said solid carrier material, **characterised in that** the one or more water-soluble acidic components absorbed into and/or onto the solid carrier material constitute at least 50% by weight of the powdered acid-loaded carrier particles.

2. The powdered acid-loaded carrier material according to claim 1 wherein the solid carrier material is pregelatinised starch selected from the group consisting of pregelatinised non-granular starch material, pregelatinised granular starch, partially gelatinised starch, and mixtures of two or more thereof, preferably pregelatinised non-granular starch.

3. The powdered acid-loaded carrier material according to claim 1 or 2 wherein the pregelatinised, non-granular starch material is consisting of flake-shaped starch particles, wherein the size distribution of the starch particles is such that at least 50% by weight of the starch particles have a particle size of between 100 µm and 375 µm.

4. The powdered acid-loaded carrier material according to anyone of claims 1 to 3 wherein the pregelatinised, non-granular starch material is having BET specific surface area less than or equal to 0.5 m²/g.

5. The powdered acid-loaded carrier material according to claim 1 wherein the puffed starch is having a loose bulk density of less than 150 g/l, preferably less than 120 g/l, more preferably less than 100 g/l.

6. The powdered acid-loaded carrier material according to anyone of claims 1 to 5 wherein the water-soluble acidic component is selected from the group consisting of acetic acid, formic acid, propionic acid, sulphuric acid, citric acid, ascorbic acid, salycilic acid, malic acid, sorbic acid, lactic acid, tartaric acid, phosphoric acid, fumaric acid, leavening acids and mixtures of two or more thereof.

7. A process for preparing a powdered acid-loaded starch material according to anyone of claims 1 to 6, comprising the steps of:
a. providing a solid carrier material being a pregelatinised starch selected from the group consisting of pregelatinised non-granular starch material, pregelatinised granular starch, partially gelatinized starch, puffed starch and mixtures of two or more thereof,
b. loading one or more water-soluble liquid acidic components into and/or onto the solid carrier material provided in step (a) by applying one or more water-soluble liquid acidic components to the carrier material under agitation, wherein said acidic components are absorbed into and/or onto said carrier material at an amount constituting at least 50% by weight of the powdered acid loaded carrier material.
**characterized in that** the one or more water-soluble liquid acidic components are in the form of a concentrated aqueous solution of acid containing less than 10% of water, or in the form of a non-aqueous solution containing ethanol or methanol, or in the form of a melted acid.

8. The process of claim 7, wherein flowing agents and/or anti-caking agents are added to the acid-loaded carrier particles.

9. Use of a powdered acid-loaded carrier material according to anyone of claims 1 to 6 for use in food and animal feed products, pharmaceuticals, nutraceuticals, agrochemicals, cleaning agents, home care products, industrial products, and cosmetic or personal care products.

10. Use according to claim 9 wherein the food products are selected from snacks, instant soups, seasoning, sauces, dry mixes for sauce preparations, dressings, marinades, dips, meat products, bakery products, baking powders, confectionery, dairy products, dried and/or freeze-dried vegetables.

11. A snack flavoured with the powdered acid-loaded carrier material according to anyone of claim 1 to 6, and wherein the powdered acid-loaded carrier material is present in an amount of 0.5-10 %.

## Patentansprüche

1. Pulverisiertes, säurebeladenes Trägermaterial, umfassend eine oder mehrere wasserlösliche, flüssige, saure Komponenten und ein festes Trägermaterial, das eine prägelatinisierte Stärke ist ausgewählt aus der Gruppe bestehend aus prägelatinisiertem, nichtgranulösem Stärkematerial, prägelatinisierter, granulöser Stärke, teilweise gelatinisierter Stärke, aufgequollener Stärke und Mischungen von zwei oder mehreren davon und wobei die wasserlösliche, flüssige, saure Komponente in und/oder auf das feste Trägermaterial absorbiert ist, **dadurch gekennzeichnet, dass** die eine oder mehreren wasserlöslichen, sauren Komponenten, das/die in und/oder auf das feste Trägermaterial absorbiert ist/sind, mindestens 50 Gew.-% der pulverisierten, säurebeladenen Trägerteilchen bildet/bilden.

2. Pulverisiertes, säurebeladenes Trägermaterial nach Anspruch 1, wobei das feste Trägermaterial prägelatinisierte Stärke ist ausgewählt aus der Gruppe bestehend aus prägelatinisiertem, nichtgranulösem Stärkematerial, prägelatinisierter, granulöser Stärke, teilweise gelatinisierter Stärke und Mischungen von zwei oder mehreren davon, bevorzugt prägelatinisierter nichtgranulöser Stärke.

3. Pulverisiertes, säurebeladenes Trägermaterial nach Anspruch 1 oder 2, wobei das prägelatinisierte, nichtgranulöse Stärkematerial aus flockenförmigen Stärketeilchen besteht, wobei die Größenverteilung der Stärketeilchen derart ist, dass mindestens 50 Gew.-% der Stärketeilchen eine Teilchengröße zwischen 100 µm und 375 µm aufweisen.

4. Pulverisiertes, säurebeladenes Trägermaterial nach einem der Ansprüche 1 bis 3, wobei das prägelatinisierte, nichtgranulöse Stärkematerial einen spezifischen BET-Oberflächenbereich von weniger als oder gleich 0,5 m²/g aufweist.

5. Pulverisiertes, säurebeladenes Trägermaterial nach Anspruch 1, wobei die aufgequollene Stärke eine lose Schüttdichte von weniger als 150 g/l, bevorzugt weniger als 120 g/l, noch bevorzugter weniger als 100 g/l aufweist.

6. Pulverisiertes, säurebeladenes Trägermaterial nach einem der Ansprüche 1 bis 5, wobei die wasserlösliche, saure Komponente aus der Gruppe ausgewählt ist bestehend aus Essigsäure, Ameisensäure, Propionsäure, Schwefelsäure, Zitronensäure, Ascorbinsäure, Salicylsäure, Apfelsäure, Sorbinsäure, Milchsäure, Weinsäure, Phosphorsäure, Fumarsäure, Gärsäuren und Mischungen von zwei oder mehreren davon.

7. Verfahren zur Herstellung eines pulverisierten, säurebeladenen Trägermaterials nach einem der Ansprüche 1 bis 6, umfassend die Schritte des:
a. Bereitstellens eines festen Trägermaterials, das eine prägelatinisierte Stärke ist ausgewählt aus der Gruppe bestehend aus prägelatinisiertem. nichtgranulösem Stärkematerial, prägelatinisierter, granulöser Stärke, teilweise gelatinisierter Stärke, aufgequollener Stärke und Mischungen von zwei oder mehreren davon,
b. Ladens einer oder mehrerer wasserlöslicher, flüssiger, saurer Komponenten in und/oder auf das feste Trägermaterial, das in Schritt (a) bereitgestellt worden ist, durch Aufbringen einer oder mehrerer wasserlöslicher, flüssiger, saurer Komponenten auf das Trägermaterial unter Bewegung, wobei die sauren Komponenten in und/oder auf das Trägermaterial in einer Menge absorbiert werden, die mindestens 50 Gew.-% des pulverisierten, säurebeladenen Trägermaterials bildet,
**dadurch gekennzeichnet, dass** eine oder mehrere wasserlösliche, flüssige, saure Komponenten in Form einer konzentrierten, wässrigen Lösung von Säure, die weniger als 10 % Wasser enthält, oder in Form einer nichtwässrigen Lösung, die Ethanol oder Methanol enthält, oder in Form einer geschmolzenen Säure vorliegt/vorliegen.

8. Verfahren nach Anspruch 7, wobei Fließmittel und/oder Antiagglomerationsmittel den säurebeladenen Trägerteilchen zugegeben werden.

9. Verwendung eines pulverisierten, säurebeladenen Trägermaterials nach einem der Ansprüche 1 bis 6 zur Verwendung in Nahrungsmittel- und Tierfutterprodukten, Pharmazeutika, Neutrazeutika, Agrochemikalien, Reinigungsmitteln, Heimpflegeprodukten, industriellen Produkten und Kosmetika oder Körperpflegeprodukten.

10. Verwendung nach Anspruch 9, wobei die Nahrungsmittelprodukte unter Snacks, Fertigsuppen, Würzmitteln, Saucen, Trockenmischungen für Saucenpräparate, Salatsoßen, Marinaden, Tunken, Fleischprodukten, Bäckerwaren, Backpulvern, Süßwaren, Molkereiprodukten, Trockengemüsen und/oder tiefgefrorenen Gemüsen ausgewählt werden.

11. Snack, der mit dem pulverisierten, säurebeladenen Trägermaterial nach einem der Ansprüche 1 bis 6 aromatisiert ist und wobei das pulverisierte, säurebeladene Trägermaterial in einer Menge von 0,5 - 10 % vorliegt.

## Revendications

1. Matériau support en poudre chargé d'acide, comprenant un ou plusieurs composants acides liquides hydrosolubles et un matériau support solide qui est un amidon prégélatinisé sélectionné à partir du groupe constitué par une matière d'amidon non granulaire prégélatinisé, de l'amidon granulaire prégélatinisé, de l'amidon partiellement gélatinisé, de l'amidon soufflé et des mélanges de deux ou plusieurs de ceux-ci, et dans laquelle le composant acide liquide hydrosoluble est absorbé dans et/ou sur ledit matériau support solide, **caractérisé en ce que** le ou les plusieurs composants acides hydrosolubles absorbés dans et/ou sur le matériau support solide constituent au moins 50 % en poids des particules de poudre support chargés d'acide.

2. Matériau support en poudre chargé d'acide selon la revendication 1, dans lequel le matériau support solide est de l'amidon prégélatinisé sélectionné à partir du groupe constitué par un matériau d'amidon non granulaire prégélatinisé, de l'amidon granulaire prégélatinisé, de l'amidon partiellement gélatinisé et des mélanges de deux ou plusieurs de ceux-ci, de préférence de l'amidon non granulaire prégélatinisé.

3. Matériau support en poudre chargé d'acide selon la revendication 1 ou 2, dans lequel le matériau d'amidon non granulaire prégélatinisé consiste en particules d'amidon en forme de flocons, dans lesquelles la distribution de taille des particules d'amidon est telle qu'au moins 50 % en poids des particules d'amidon ont une taille de particule comprise entre 100 µm et 375 µm.

4. Matériau support en poudre chargé d'acide selon l'une quelconque des revendications 1 à 3, dans lequel le matériau d'amidon non granulaire prégélatinisé a une superficie spécifique BET inférieure ou égale à 0,5 m²/g.

5. Matériau support en poudre chargé d'acide selon la revendication 1, dans lequel l'amidon soufflé a une masse volumique en vrac lâche inférieure à 150 g/l, de préférence inférieure à 120 g/l, plus de préférence inférieure à 100 g/l.

6. Matériau support en poudre chargé d'acide selon l'une quelconque des revendications 1 à 5, dans lequel le composant acide hydrosoluble est sélectionné à partir du groupe constitué par l'acide acétique, l'acide formique, l'acide propionique, l'acide sulfurique, l'acide citrique, l'acide ascorbique, l'acide salycilique, l'acide malique, l'acide sorbique, l'acide lactique, l'acide tartrique, l'acide phosphorique, l'acide fumarique, des acides de levage et des mélanges de deux ou plusieurs de ceux-ci.

7. Processus pour préparer un matériau d'amidon en poudre chargé d'acide selon l'une quelconque des revendications 1 à 6, comprenant les étapes consistant à :
a. fournir un matériau support solide qui est un amidon prégélatinisé sélectionné à partir du groupe constitué par un matériau d'amidon non granulaire prégélatinisé, de l'amidon granulaire prégélatinisé, de l'amidon partiellement gélatinisé, de l'amidon soufflé et des mélanges de deux ou plusieurs de ceux-ci,
b. charger un ou plusieurs composants acides liquides hydrosolubles dans et/ou sur le matériau support solide fourni dans l'étape (a) en appliquant un ou plusieurs composants acides liquides hydrosolubles au matériau support sous agitation, dans lequel lesdits composants acides sont absorbés dans et/ou sur ledit matériau support en une quantité constituant au moins 50 % en poids du matériau support en poudre chargé d'acide,
**caractérisé en ce que** le ou les plusieurs composants acides liquides hydrosolubles sont sous la forme d'une solution aqueuse concentrée d'acide contenant moins de 10 % d'eau, ou sous la forme d'une solution non aqueuse contenant de l'éthanol ou du méthanol, ou sous la forme d'un acide fondu.

8. Processus selon la revendication 7, dans lequel des agents fluidifiants et/ou des agents antiagglomérants sont ajoutés aux particules support chargés d'acide.

9. Utilisation d'un matériau support en poudre chargé d'acide selon l'une quelconque des revendications 1 à 6 à utiliser dans des produits d'alimentation et de fourrage, des produits pharmaceutiques, des produits nutraceutiques, des produits agrochimiques, des agents de nettoyage, des produits d'hygiène domestique, des produits industriels et des produits cosmétiques ou d'hygiène personnelle.

10. Utilisation selon la revendication 9, dans laquelle les produits alimentaires sont sélectionnés parmi les encas, les potages instantanés, les assaisonnements, les sauces, des mélanges secs pour des préparations de sauce, des accompagnements, des marinades, des sauces pour apéritif, des produits carnés, des produits de boulangerie, des levures, de la confiserie, des produits laitiers, des légumes séchés et/ou lyophilisés.

11. Encas aromatisé avec le matériau support en poudre chargé d'acide selon l'une quelconque des revendications 1 à 6 et dans lequel le matériau support en poudre chargé d'acide est présent en une quantité de 0,5 à 10 %.
